# EUROPEAN PATENT APPLICATION

(11) **EP 2 923 763 A1**
(43) Date of publication of application: **30.09.2015**
(21) Application number: 15160772.8
(22) Date of filing: 25.03.2015
(51) Int. Cl.: B01L 3/00, B01L 7/00

(54) **BIOCHIP**

(30) Priority: 27.03.2014 JP 2014066849
(71) Applicant: Seiko Epson Corporation, Shinjuku-ku Tokyo 163-0811 (JP)
(72) Inventor: Yamaguchi, Akemi, Nagano, 392-8502 (JP)
(74) Representative: Hoffmann Eitle

(57) **Abstract**

A biochip comprising:
a vessel;
a liquid which is contained in the vessel; and
an additive which is a carbinol-modified silicone resin, a carboxyl-modified silicone resin, an amino-modified silicone resin, a polyether-modified silicone resin, a silanol-modified silicone resin, or a fluoro-modified silicone resin,

wherein a liquid droplet of a reaction mixture which (i) has a different specific gravity from that of the liquid; (ii) is immiscible with the liquid and (iii) contains a surfactant, can move in the longitudinal direction of the vessel when placed in the vessel.

## Description

### BACKGROUND

### 1. Technical Field

The present invention relates to a biochip.

### 2. Related Art

In recent years, as a result of development of technologies utilizing genes, medical treatments utilizing genes such as gene diagnosis and gene therapy are drawing attention. In addition, many methods utilizing genes in determination of breed varieties or breed improvement have also been developed in agricultural and livestock industries. As technologies for utilizing genes, nucleic acid amplification technologies such as a PCR (Polymerase Chain Reaction) method are widely used. Nowadays, a PCR method has become an indispensable technology for elucidation of information on biological materials.

In the PCR method, a method in which a reaction is performed using a vessel for performing a biochemical reaction called a tube or a chip (hereinafter referred to as "biochip") is generally used. However, in the method of the related art, there are problems that a large amount of a reagent or the like is needed, an apparatus is complicated in order to realize a thermal cycle required for the reaction, and it takes time to perform the reaction. Therefore, a biochip or a reaction apparatus for performing PCR in a short time using a very small amount of a reagent or a specimen has been demanded.

In order to solve such a problem, JP-A-2009-136250 (PTL 1) discloses a biochip and an apparatus for performing a reaction through a thermal cycle by allowing a reaction mixture contained in the form of a liquid droplet to move reciprocatingly in a tube filled with a liquid (such as a mineral oil) which is immiscible with the reaction mixture and has a different specific gravity from that of the reaction mixture.

However, in the case where the biochip disclosed in PTL 1 is used in an application in which an amplification product is detected by measuring fluorescence from the outside of a vessel, it is necessary to form the vessel from a transparent material. As the transparent material, a resin or a heat-resistant glass can be used, however, these materials are easily charged with electricity by friction or the like. It is possible to prevent static electricity by performing a hydrophilic treatment on an inner surface of the vessel, however, the reaction mixture which is an aqueous solution adheres to the vessel to hinder the movement of the reaction mixture. Due to this, it was difficult to adopt a hydrophilic treatment for a biochip.

On the other hand, as the liquid which is immiscible with the reaction mixture and has a different specific gravity from that of the reaction mixture, a silicone oil or a mineral oil can be used from the viewpoint of stability against heat or the reaction mixture. However, these oils are generally insulating materials, and therefore, a liquid droplet of the reaction mixture introduced into the oil is easily polarized. Due to this, when a vessel formed from a transparent material is filled with such an oil and the reaction mixture is introduced into the oil, an electric field is generated between the reaction mixture and the vessel, and the reaction mixture is attracted and adheres to the inner wall of the vessel or floats in the oil due to repulsion in some cases. If PCR of a system in which the reaction mixture is allowed to move by the gravitational force in the biochip as described in PTL 1 (hereinafter, in this specification, this system is referred to as "elevating-type system") is performed in such a state, the reaction mixture does not appropriately move, and therefore, a desired thermal cycle cannot be performed in some cases.

Therefore, JP-A-2012-125169 (PTL 2) discloses a biochip which is configured such that when a vessel filled with a liquid immiscible with a reaction mixture is used, the volume resistivity of the liquid is set to more than 0 Ω·cm and 5×10¹³ Ω·cm or less in order to eliminate the electric charge generated in the biochip and subject the reaction mixture to a stable thermal cycle.

However, even if static electricity is prevented as described in PTL 2, a liquid droplet still adheres to the vessel, and therefore, the liquid droplet cannot appropriately move in the oil in some cases.

### SUMMARY

An advantage of some aspects of the invention is to provide a biochip in which a reaction mixture is prevented from adhering to the inner wall of a vessel so that the reaction mixture can appropriately move in the vessel.

The invention can be implemented as the following aspects.
A biochip according to an aspect of the invention is a biochip comprising:
a vessel;
a liquid which is contained in the vessel; and
an additive which is a carbinol-modified silicone resin, a carboxyl-modified silicone resin, an amino-modified silicone resin, a polyether-modified silicone resin, a silanol-modified silicone resin, or a fluoro-modified silicone resin,
wherein a liquid droplet of a reaction mixture which (i) has a different specific gravity from that of the liquid contained in the vessel; (ii) is immiscible with the liquid contained in the vessel and (iii) contains a surfactant, can move in the longitudinal direction of the vessel when placed in the vessel.

The liquid may be a mineral oil or a silicone oil. The additive may be any of X-22-160AS, X-22-3701E, KF-857, KF-859, KF-862, KF-867, KF-6017, KF-8005 (Shin-Etsu Silicone Co., Ltd.), SR1000, SS4230, SS4267, YR3370, XS66-C1191, TSF4703, TSF4708, XF42-C5196, and XF42-C5197 (Momentive Performance Materials, Inc.). The biochip may comprise a liquid droplet of a reaction mixture. The reaction mixture may contain a reagent for a nucleic acid amplification reaction. The concentration of the additive may be 1% (v/v) or more and 50% (v/v) or less. The vessel may be made of polypropylene. The surfactant may be NP-40, Triton X-100, or Tween 20.

A nucleic acid amplification apparatus according to another aspect of the invention includes any one of the above-described biochips, which is fitted therein.

According to the aspects of the invention, a biochip in which a reaction mixture is prevented from adhering to the inner wall of a vessel so that the reaction mixture can appropriately move in the vessel can be provided.

### BRIEF DESCRIPTION OF THE DRAWINGS

The invention will be described with reference to the accompanying drawings, wherein like numbers reference like elements.
FIG. 1 is a cross-sectional view of a nucleic acid amplification reaction chip according to an embodiment of the invention. The arrow g indicates the direction of the gravitational force.
FIGS. 2A and 2B are perspective views of an elevating-type PCR apparatus according to one embodiment of the invention. FIG. 2A shows a state in which a lid is closed, and FIG. 2B shows a state in which the lid is opened.
FIG. 3 is an exploded perspective view of a main body of the elevating-type PCR apparatus according to one embodiment of the invention.
FIGS. 4A and 4B are cross-sectional views schematically showing the cross section taken along the line A-A in FIG. 2A of the main body of the elevating-type PCR apparatus according to one embodiment of the invention. FIG. 4A shows a first arrangement, and FIG. 4B shows a second arrangement.

### DESCRIPTION OF EXEMPLARY EMBODIMENTS

The object, characteristics, and advantages of the invention as well as the idea thereof will be apparent to those skilled in the art from the description given herein, and the invention can be easily reproduced by those skilled in the art based on the description given herein. It is to be understood that the embodiments, specific examples, etc. of the invention described below are to be taken as preferred embodiments of the invention, and are presented for illustrative or explanatory purposes and are not intended to limit the invention. It is further apparent to those skilled in the art that various changes and modifications may be made based on the description given herein within the intent and scope of the invention disclosed herein.

### (1) Biochip

The biochip according to the invention is a biochip comprising:
a vessel;
a liquid which is contained in the vessel; and
an additive which is a carbinol-modified silicone resin, a carboxyl-modified silicone resin, an amino-modified silicone resin, a polyether-modified silicone resin, a silanol-modified silicone resin, or a fluoro-modified silicone resin,
wherein a liquid droplet of a reaction mixture which (i) has a different specific gravity from that of the liquid; (ii) is immiscible with the liquid and (iii) contains a surfactant, can move in the longitudinal direction of the vessel when placed in the vessel.. In this biochip, a risk that the reaction mixture in the form of a liquid droplet adheres to the inner wall of the vessel is reduced, so that the reaction mixture easily moves in the longitudinal direction of the vessel. Hereinafter, the configuration of the biochip will be described in detail.

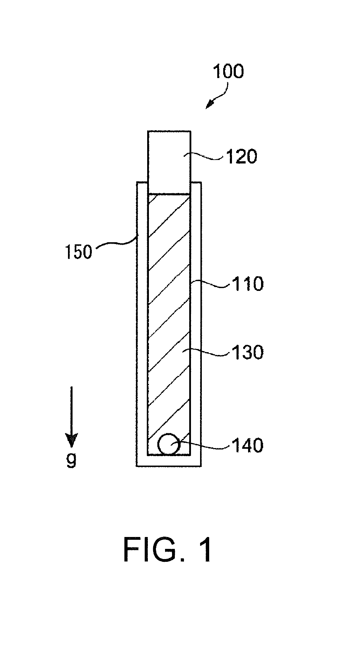

FIG. 1 is a cross-sectional view of a biochip 100. FIG. 1 shows a state in which a reaction mixture is placed in the biochip.

The biochip 100 according to the invention is configured to include a vessel 150 and a sealing section 120. The size and shape of the biochip 100 are not particularly limited, but for example, the biochip may be designed in consideration of at least one of the amount of an oil 130 which is immiscible with a reaction mixture 140, the thermal conductivity of the oil 130, the shape of the vessel 150 and the sealing section 120, and the ease of handling.

The vessel 150 of the biochip 100 can be formed from a transparent material. According to this, the movement of the reaction mixture 140 in the vessel 150 can be observed from the outside of the biochip 100, or the biochip 100 can be used in an application in which the measurement or the like is performed from the outside of the vessel 150 such as real-time PCR. The term "transparent" as used herein refers to a condition in which the visibility to such an extent that the reaction mixture 140 in the vessel 150 can be observed from the outside of the vessel 150 can be ensured, and it is not necessary that the entire biochip 100 should be transparent as long as this condition is met.

The application of the biochip 100 is not particularly limited, however, for example, in the case where the biochip 100 is used in an application with a fluorescence measurement such as real-time PCR, the vessel 150 is desirably formed from a material with a low autofluorescence. The vessel 150 is preferably formed from a material which can withstand heating in PCR. Further, the material of the vessel 150 is preferably a material, on which nucleic acids or proteins are less adsorbed, and which does not inhibit the enzymatic reaction by a polymerase or the like. The material which satisfies these conditions is not particularly limited, and for example, polypropylene, polyethylene, a cycloolefin polymer (for example, ZEONEX (registered trademark) 480R), a heat-resistant glass (for example, PYREX (registered trademark) glass), or the like, or a composite material thereof may be used, however, polypropylene is preferred.

In the biochip 100 shown in FIG. 1, the vessel 150 is formed into a cylindrical shape, and the direction of the center axis (the vertical direction in Fig. 1) coincides with the longitudinal direction. The vessel 150 used here is preferably a tube and may be a tube for a microcentrifuge or a tube designed for PCR. Since the vessel 150 has a shape with a longitudinal direction, in other words, an elongated shape, for example, in the case where the temperature of the biochip 100 is controlled so that regions having different temperatures are formed in the oil 130 in the vessel 150 using an elevating-type thermal cycler, which will be described later, the distance between the regions having different temperatures is easily increased, According to this, it becomes easy to control the temperature of the oil 130 to be different from region to region in the vessel 150, and therefore, a thermal cycle suitable for PCR can be realized. The "elevating-type thermal cycler" is an apparatus which performs a thermal cycle by forming at least two temperature regions in an oil filled in the vessel 150 and allowing the reaction mixture 140 which is phase-separated from the oil to move reciprocatingly between these temperature regions.

The shape of the vessel 150 is not particularly limited as long as it has a longitudinal direction, however, in the case where the vessel 150 is used for elevating-type PCR, it is preferred that the shape is a substantially cylindrical shape and the ratio of the inner diameter D to the length L in the longitudinal direction is in the range of 1:5 to 5:20. It is more preferred that the inner diameter D is from 1.5 to 2 mm, and the length L is from 10 to 20 mm.

The vessel 150 has an opening section and the sealing section 120 which seals the opening section, and in the vessel 150, the reaction mixture 140 and the liquid 130 which has a different specific gravity from that of the reaction mixture 140 and is phase-separated from the reaction mixture 140 are contained. It is preferred that in the case where the opening section is sealed by the sealing section 120, air does not remain in the vessel 150. It is because if an air bubble remains in the vessel 150, the movement of the reaction mixture 140 may be hindered. The sealing section 120 can be formed from the same material as that of the vessel 150. The structure of the sealing section 120 may be any as long as it can hermetically seal the vessel 150, and can be a structure of, for example, a screw cap, a plug, an inlay, or the like. In FIG. 1, the sealing section 120 has a structure of a screw cap.

The reaction to which the reaction mixture 140 is subjected is not particularly limited, however, the reaction mixture 140 may be subjected to a nucleic acid amplification reaction and may contain a reagent for a nucleic acid amplification reaction and may also contain a target nucleic acid to be amplified. Examples of the target nucleic acid include a DNA prepared from a specimen such as blood, urine, saliva, spinal fluid, or a tissue and a cDNA obtained by reverse transcription of an RNA prepared from any of the above specimens. The reagent for a nucleic acid amplification reaction may contain a primer for amplifying a target nucleic acid, a buffer, a polymerase, dNTPs, MgCl₂, a fluorescent probe for detecting an amplification product of the target nucleic acid, and the like. The DNA polymerase is not particularly limited, but is preferably a heat-resistant enzyme or an enzyme for use in PCR. There are a great number of commercially available products, for example, Taq polymerase, Tfi polymerase, Tth polymerase, modified forms thereof, and the like, however, a DNA polymerase capable of performing hot start PCR is preferred. The concentration of dNTPs or a salt may be set to a concentration suitable for the enzyme to be used, however, the concentration of dNTPs may be set to generally 10 to 1000 µM, preferably 100 to 500 µM, the concentration of Mg²⁺ may be set to generally 1 to 100 mM, preferably 5 to 10 mM, and the concentration of Cl⁻ may be set to 1 to 2000 mM, preferably 200 to 700 mM. The total ion concentration is not particularly limited, but may be higher than 50 mM, and is preferably higher than 100 mM, more preferably higher than 120 mM, further more preferably higher than 150 mM, still further more preferably higher than 200 mM. The upper limit thereof is preferably 500 mM or less, more preferably 300 mM or less, further more preferably 200 mM or less. Each oligonucleotide for the primer is used at 0.1 to 10 µM, preferably 0.1 to 1 µM.

The reaction mixture 140 may further contain a surfactant. The surfactant is not particularly limited, however, examples thereof include NP-40, Triton X-100, and Tween 20. The concentration of the surfactant is not particularly limited, but is preferably a concentration which does not inhibit the nucleic acid amplification reaction, and may be from 0.001% to 0.1%, and is preferably from 0.002% to 0.02%, most preferably from 0.005% to 0.01%. The surfactant may be a carry-over from a stock solution of the enzyme described above, however, a surfactant solution may be added to the reaction mixture 140 separately therefrom.

By using a liquid which is immiscible with the reaction mixture 140 as the liquid 130, when the reaction mixture 140 is placed in the vessel 150, the reaction mixture 140 and the liquid 130 are phase-separated from each other, and therefore, the reaction mixture 140 can be formed into a liquid droplet in the liquid 130. In this manner, the reaction mixture 140 is maintained in the form of a liquid droplet in the liquid 130.

The liquid 130 is preferably a liquid having a specific gravity smaller than that of the reaction mixture 140. In this case, when the reaction mixture 140 is placed in the liquid 130, the liquid droplet of the reaction mixture 140 has a specific gravity larger than that of the liquid 130, and therefore moves in the direction in which the gravitational force acts by the action of the gravity. Further, the liquid 130 may be a liquid having a specific gravity larger than that of the reaction mixture 140. In this case, the liquid droplet of the reaction mixture 140 has a specific gravity smaller than that of the liquid 130, and therefore moves in the direction opposite to the direction in which the gravitational force acts by the action of the gravity.

The liquid 130 preferably contains an oil, and for example, a silicone oil or a mineral oil can be used. Here, the "silicone" means an oiligomer or a polymer having a siloxane bond as a main skeleton. In this specification, among silicones, a silicone in the form of a liquid in a temperature range in which the silicone is used in a thermal cycling process is particularly referred to as "silicone oil". Further, in this specification, an oil which is purified from petroleum and is in the form of a liquid in a temperature range in which the oil is used in a thermal cycling process is referred to as "mineral oil". These oils have high stability against heat, and for example, products having a viscosity of 5×10³ Nsm⁻² or less are also easily available, and therefore, these oils are preferred for use in elevating-type PCR.

Examples of the silicone oil include dimethyl silicone oils such as KF-96L-0.65cs, KF-96L-1cs, KF-96L-2cs, KF-96L-5cs (Shin-Etsu Silicone Co., Ltd.), SH200 C FLUID 5 CS (Dow Corning Toray Co., Ltd.), TSF451-5A, and TSF451-10 (Momentive Performance Materials Japan LLC Company Ltd.). Examples of the mineral oil include oils containing alkane having about 14 to 20 carbon atoms as a principal component, and specific examples thereof include n-tetradecane, n-pentadecane, n-hexadecane, n-heptadecane, n-octadecane, n-nonadecane, and n-tetracosane.

The liquid 130 contains an additive. The additive is a carbinol-modified silicone resin, a carboxyl-modified silicone resin, an amino-modified silicone resin, a polyether-modified silicone resin, a silanol-modified silicone resin, or a fluoro-modified silicone resin, and for example, a modified silicone oil such as X-22-160AS, X-22-3701E, KF-857, KF-859, KF-862, KF-867, KF-6017, or KF-8005 (Shin-Etsu Silicone Co., Ltd.), a silicone resin such as SR1000, SS4230, SS4267, or YR3370 (Momentive Performance Materials, Inc.), a fluoro-modified silicone resin such as XS66-C1191 (Momentive Performance Materials, Inc.), or the like, and other than these, a modified silicone oil such as TSF4703, TSF4708, XF42-C5196, or XF42-C5197 (Momentive Performance Materials, Inc.) can be used. The concentration of the additive is not particularly limited, but can be determined in consideration of the structure, material, shape, or the like of the vessel. For example, the concentration thereof is set to preferably 1% (v/v) or more and 50% (v/v) or less, more preferably 2% (v/v) or more and 20% (v/v) or less, further more preferably 5% (v/v) - all these values are based on the total amount of liquid plus additive.

### (2) Structure of Elevating-Type Nucleic Acid Amplification Reaction Apparatus

In this embodiment, as the biochip to be used for performing a nucleic acid amplification reaction, a nucleic acid amplification reaction tube 100 in the form of a tube is used. Hereinafter, one example of an elevating-type nucleic acid amplification reaction apparatus (hereinafter also referred to as "elevating-type PCR apparatus") suitable for the nucleic acid amplification reaction tube 100 will be described in detail.

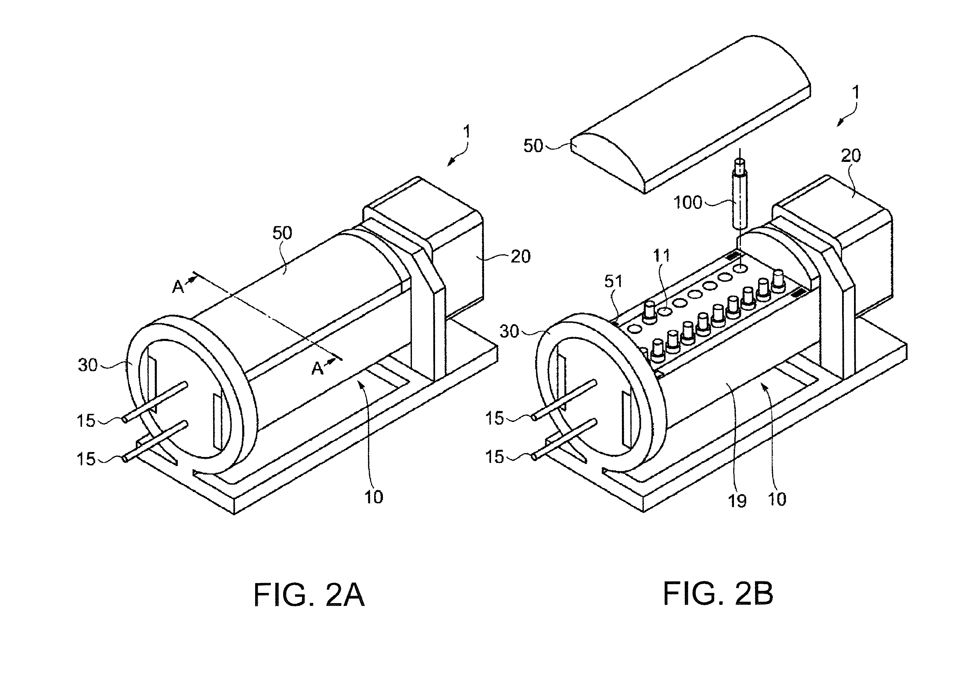

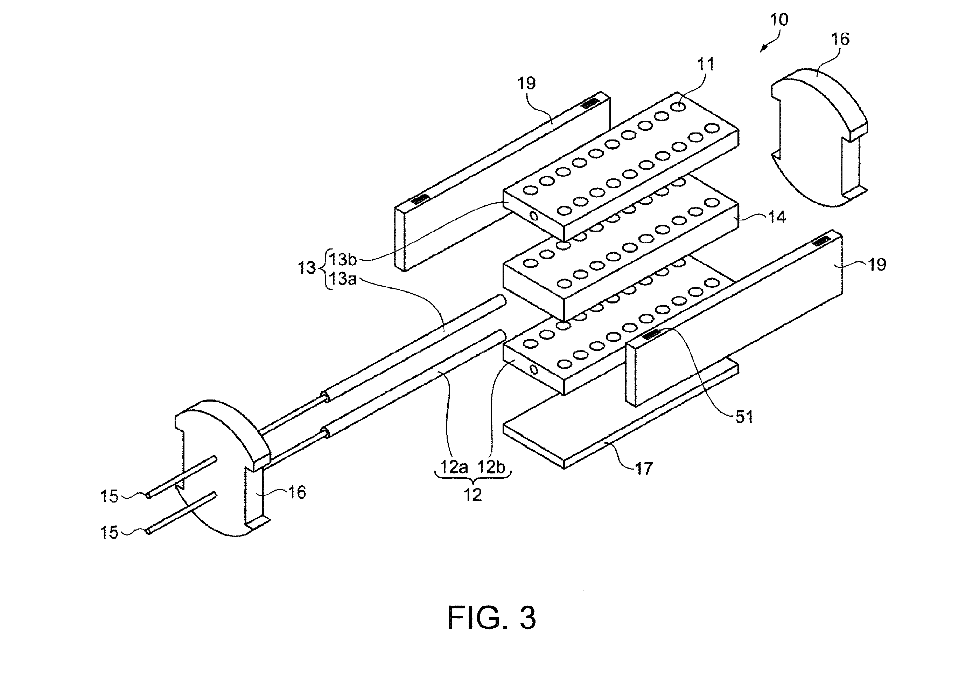

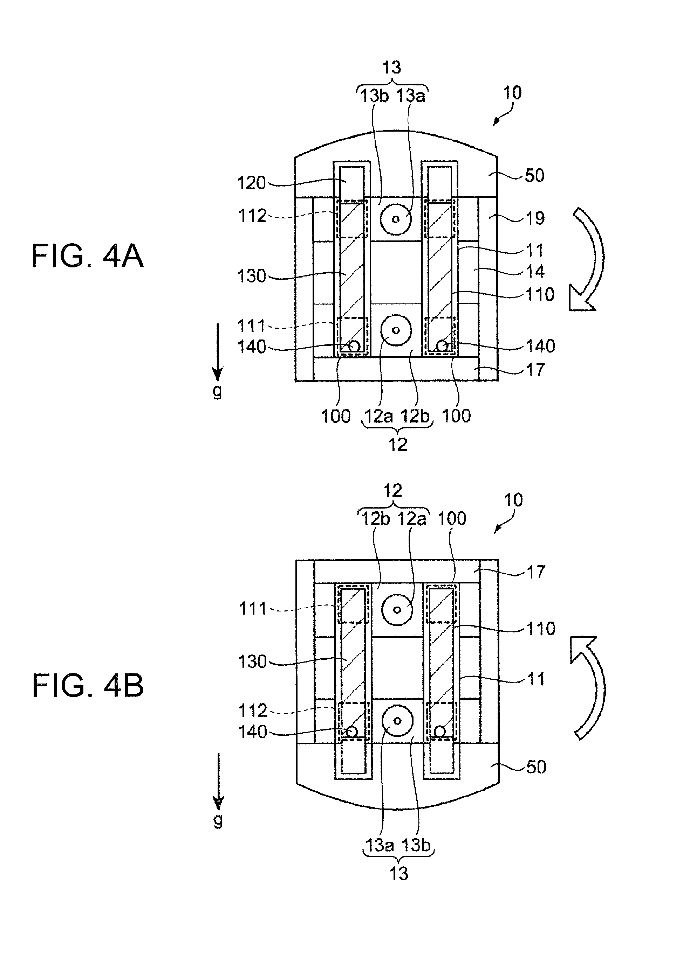

FIGS. 2A and 2B show one example of an elevating-type PCR apparatus 1. FIG. 2A shows a state in which a lid 50 of the elevating-type PCR apparatus 1 is closed, and FIG. 2B shows a state in which the lid 50 of the elevating-type PCR apparatus 1 is opened and the nucleic acid amplification reaction tube 100 is fitted in a fitting section 11. FIG. 3 is an exploded perspective view of a main body 10 of the elevating-type PCR apparatus 1 according to the embodiment. FIG. 4A is a cross-sectional view schematically showing the cross section taken along the line A-A in FIG. 2A of the main body 10 of the elevating-type PCR apparatus 1 according to the embodiment.

This elevating-type PCR apparatus 1 includes the main body 10 and a driving mechanism 20 as shown in FIG. 2A. As shown in FIG. 3, the main body 10 includes the fitting section 11, a first heating section 12, and a second heating section 13. A spacer 14 is provided between the first heating section 12 and the second heating section 13. In the main body 10 of this embodiment, the first heating section 12 is disposed on the side of a bottom plate 17, and the second heating section 13 is disposed on the side of the lid 50. In the main body 10 of this embodiment, the first heating section 12, the second heating section 13, and the spacer 14 are fixed by a flange 16, the bottom plate 17, and a fixing plate 19.

The fitting section 11 is configured such that the nucleic acid amplification reaction tube 100, which will be described later, is fitted therein. As shown in FIG. 2B and FIG. 3, the fitting section 11 of this embodiment has a slot structure in which the nucleic acid amplification reaction tube 100 is inserted and fitted, and is configured such that the nucleic acid amplification reaction tube 100 is inserted into a hole penetrating a first heat block 12b of the first heating section 12, the spacer 14, and a second heat block 13b of the second heating section 13. The number of the fitting sections 11 may be more than one, and in the example shown in FIG. 2B, twenty fitting sections 11 are provided for the main body 10.

This elevating-type PCR apparatus 1 includes a structure in which the nucleic acid amplification reaction tube 100 is held at a predetermined position with respect to the first heating section 12 and the second heating section 13. More specifically, as shown in FIGS. 4A and 4B, in a flow channel 110 constituting the nucleic acid amplification reaction tube 100, which will be described later, a first region 111 can be heated by the first heating section 12 and a second region 112 can be heated by the second heating section 13. In this embodiment, a structure that defines the position of the nucleic acid amplification reaction tube 100 is the bottom plate 17, and as shown in FIG. 4A, by inserting the nucleic acid amplification reaction tube 100 to a position in contact with the bottom plate 17, the nucleic acid amplification reaction tube 100 can be held at a predetermined position with respect to the first heating section 12 and the second heating section 13.

When the nucleic acid amplification reaction tube 100 is fitted in the fitting section 11, the first heating section 12 heats the first region 111 of the nucleic acid amplification reaction tube 100, which will be described later, to a first temperature. In the example shown in FIG. 4A, in the main body 10, the first heating section 12 is disposed at a position so as to heat the first region 111 of the nucleic acid amplification reaction tube 100.

The first heating section 12 may include a mechanism that generates heat and a member that transfers the generated heat to the nucleic acid amplification reaction tube 100. In the example shown in FIG. 3, the first heating section 12 includes a first heater 12a and a first heat block 12b. In this embodiment, the first heater 12a is a cartridge heater, and is connected to an external power source (not shown) through a conductive wire 15. The first heater 12a is inserted into the first heat block 12b, and the first heat block 12b is heated by heat generated by the first heater 12a. The first heat block 12b is a member that transfers heat generated by the first heater 12a to the nucleic acid amplification reaction tube 100. In this embodiment, the first heat block 12b is a block made of aluminum.

When the nucleic acid amplification reaction tube 100 is fitted in the fitting section 11, the second heating section 13 heats the second region 112 of the nucleic acid amplification reaction tube 100 to a second temperature different from the first temperature. In the example shown in FIG. 4A, in the main body 10, the second heating section 13 is disposed at a position so as to heat the second region 112 of the nucleic acid amplification reaction tube 100. As shown in FIG. 2, the second heating section 13 includes a second heater 13a and the second heat block 13b. The second heating section 13 is configured in the same manner as the first heating section 12 except that the region of the nucleic acid amplification reaction tube 100 to be heated and the heating temperature are different from those for the first heating section 12.

In this embodiment, the temperatures of the first heating section 12 and the second heating section 13 are controlled by a temperature sensor (not shown) and a control section (not shown), which will be described later. The temperatures of the first heating section 12 and the second heating section 13 are preferably set so as to heat the nucleic acid amplification reaction tube 100 to a desired temperature. In this embodiment, by controlling the first heating section 12 at the first temperature and the second heating section 13 at the second temperature, the first region 111 of the nucleic acid amplification reaction tube 100 can be heated to the first temperature, and the second region 112 can be heated to the second temperature. The temperature sensor in this embodiment is a thermocouple.

The driving mechanism 20 is a mechanism that drives the fitting section 11, the first heating section 12, and the second heating section 13. In this embodiment, the driving mechanism 20 includes a motor (not shown) and a drive shaft (not shown), and the drive shaft is connected to the flange 16 of the main body 10. The drive shaft in this embodiment is provided perpendicular to the longitudinal direction of the fitting section 11, and when the motor is activated, the main body 10 is rotated about the drive shaft as the axis of rotation.

The elevating-type PCR apparatus 1 of this embodiment includes the control section (not shown). The control section controls at least one of the first temperature, the second temperature, a first period, a second period, and the number of thermal cycles, which will be described later. In the case where the control section controls the first period or the second period, the control section controls the operation of the driving mechanism 20, thereby controlling the period in which the fitting section 11, the first heating section 12, and the second heating section 13 are held in a predetermined arrangement. The control section may have mechanisms different from item to item to be controlled, or may control all items collectively. However, the control section in the elevating-type PCR apparatus 1 of this embodiment is an electronic control system and controls all of the above-described items. The control section of this embodiment includes a processor such as CPU (not shown) and a storage device such as an ROM (Read Only Memory) or an RAM (Random Access Memory). In the storage device, various programs, data, etc. for controlling the above-described respective items are stored. Further, the storage device has a work area for temporarily storing data in processing, processing results, etc. of various processes.

As shown in the example of FIG. 3 and FIG. 4A, in the main body 10 of this embodiment, the spacer 14 is provided between the first heating section 12 and the second heating section 13. The spacer 14 of this embodiment is a member that holds the first heating section 12 or the second heating section 13. In this embodiment, the spacer 14 is a heat insulating material, and in the example shown in FIG. 4A, the fitting section 11 penetrates the spacer 14.

The main body 10 of this embodiment includes the fixing plate 19. The fixing plate 19 is a member that holds the fitting section 11, the first heating section 12, and the second heating section 13. In the example shown in FIG. 2B and FIG. 3, two fixing plates 19 are fitted in the flanges 16, and the first heating section 12, the second heating section 13, and the bottom plate 17 are fixed by the fixing plates 19.

The elevating-type PCR apparatus 1 of this embodiment includes the lid 50. In the example shown in FIG. 2A and FIG. 4A, the fitting section 11 is covered with the lid 50. The lid 50 may be fixed to the main body 10 by a fixing section 51. In this embodiment, the fixing section 51 is a magnet. As shown in the example of FIG. 2B and FIG. 3, a magnet is provided on a surface of the main body 10 which comes into contact with the lid 50. Although not shown in FIG. 2B and FIG. 3, a magnet is provided also for the lid 50 at a place with which the magnet of the main body 10 comes into contact. When the fitting section 11 is covered with the lid 50, the lid 50 is fixed to the main body 10 by a magnetic force.

It is preferred that the fixing plate 19, the bottom plate 17, the lid 50, and the flange 16 are formed using a heat insulating material.

### (3) Thermal Cycling Process Using Elevating-type PCR Apparatus

FIGS. 4A and 4B are cross-sectional views schematically showing the cross section taken along the line A-A in FIG. 2A of the elevating-type PCR apparatus 1. FIGS. 4A and 4B show a state in which the nucleic acid amplification reaction tube 100 is fitted in the elevating-type PCR apparatus 1. FIG. 4A shows a first arrangement, and FIG. 4B shows a second arrangement. Hereinafter, first, the nucleic acid amplification reaction tube 100 according to the embodiment will be described, and thereafter, the thermal cycling process using the elevating-type PCR apparatus 1 according to the embodiment in the case of using the nucleic acid amplification reaction tube 100 will be described.

As shown in the example of FIG. 1, the nucleic acid amplification reaction tube 100 according to the embodiment includes a flow channel 110 and a sealing section 120. The flow channel 110 is filled with a reaction mixture 140 and an oil 130 which has a specific gravity smaller than that of the reaction mixture 140 and is immiscible with the reaction mixture 140, and sealed with the sealing section 120.

The flow channel 110 is formed such that the reaction mixture 140 moves in close proximity to opposed inner walls. Here, the term "opposed inner walls" of the flow channel 110 refers to two regions of a wall surface of the flow channel 110 having an opposed positional relationship. The phrase "in close proximity to" refers to a state in which the distance between the reaction mixture 140 and the wall surface of the flow channel 110 is close, and includes a case where the reaction mixture 140 is in contact with the wall surface of the flow channel 110. Therefore, the phrase "the reaction mixture 140 moves in close proximity to opposed inner walls" refers to that "the reaction mixture 140 moves in a state of being close in distance to both of the two regions of a wall surface of the flow channel 110 having an opposed positional relationship", that is, the reaction mixture 140 moves along the opposed inner walls.

In the example shown in Fig. 1, the outer shape of the nucleic acid amplification reaction tube 100 is a cylindrical shape, and the flow channel 110 is formed in the direction of the center axis (the vertical direction in Fig. 1) therein. The shape of the flow channel 110 is a cylindrical shape having a circular cross section in the direction perpendicular to the longitudinal direction of the flow channel 110, that is, in the direction perpendicular to the direction of the movement of the reaction mixture 140 in a region in the flow channel 110 (this cross section is defined as the "cross section" of the flow channel 110). Therefore, in the nucleic acid amplification reaction tube 100 of this embodiment, the opposed inner walls of the flow channel 110 are regions including two points on the wall surface of the flow channel 110 constituting the diameter of the cross section of the flow channel 110, and the reaction mixture 140 moves in the longitudinal direction of the flow channel 110 along the opposed inner walls.

The first region 111 of the nucleic acid amplification reaction tube 100 is a partial region of the flow channel 110 which is heated to the first temperature by the first heating section 12. The second region 112 is a partial region of the flow channel 110 which is different from the first region 111 and is heated to the second temperature by the second heating section 13. In the nucleic acid amplification reaction tube 100 of this embodiment, the first region 111 is a region including one end portion in the longitudinal direction of the flow channel 110, and the second region 112 is a region including the other end portion in the longitudinal direction of the flow channel 110. In the example shown in FIGS. 4A and 4B, a region surrounded by the dotted line including an end portion on the proximal side of the sealing section 120 of the flow channel 110 is the second region 112, and a region surrounded by the dotted line including an end portion on the distal side of the sealing section 120 is the first region 111.

As shown in FIG. 1, the flow channel 110 contains the oil 130 and a liquid droplet of the reaction mixture 140. The oil 130 and the reaction mixture 140 are prepared according to the description of the above (1) Biochip.

Hereinafter, with reference to FIGS. 4A and 4B, the thermal cycling process using a thermal cycler 1 according to the embodiment will be described. In FIGS. 4A and 4B, the direction indicated by the arrow g (in the downward direction in the drawing) is the gravitational direction. In this embodiment, a case where shuttle PCR (two-stage temperature PCR) is performed will be described as an example of the thermal cycling process. The respective steps described below are shown as an example of the thermal cycling process, and according to need, the order of the steps may be changed, two or more steps may be performed continuously or concurrently, or a step may be added.

The shuttle PCR is a method of amplifying a nucleic acid in a reaction mixture by subjecting the reaction mixture to a two-stage temperature process at a high temperature and a low temperature repeatedly. In the process at a high temperature, denaturation of a double-stranded DNA occurs and in the process at a low temperature, annealing (a reaction in which a primer is bound to a single-stranded DNA) and elongation (a reaction in which a complementary strand to the DNA is formed by using the primer as a starting point) occur.

In general, in shuttle PCR, the high temperature is a temperature between 80°C and 100°C and the low temperature is a temperature between 50°C and 70°C. The processes at the respective temperatures are performed for a predetermined period, and a period of maintaining the reaction mixture at a high temperature is generally shorter than a period of maintaining the reaction mixture at a low temperature. For example, the period for the process at a high temperature may be set to about 1 to 10 seconds, and the period for the process at a low temperature may be set to about 10 to 60 seconds, or a period longer than this range may be adopted depending on the condition of the reaction.

Since the appropriate period, temperature, number of cycles (number of times of repetition of the process at a high temperature and the process at a low temperature) varies depending on the type or amount of a reagent to be used, it is preferred to determine an appropriate protocol in consideration of the type of a reagent or the amount of the reaction mixture 140 before performing the reaction.

First, the nucleic acid amplification reaction tube 100 is fitted in the fitting section 11. In this embodiment, after the reaction mixture 140 is introduced into the flow channel 110 previously filled with the oil 130, the flow channel 110 is sealed with the sealing section 120, and then, the nucleic acid amplification reaction tube 100 is fitted in the fitting section 11. The introduction of the reaction mixture 140 can be performed using a micropipette, an ink-jet dispenser, or the like. In a state in which the nucleic acid amplification reaction tube 100 is fitted in the fitting section 11, the first heating section 12 is in contact with the nucleic acid amplification reaction tube 100 at a place including the first region 111 and the second heating section 13 is in contact with the nucleic acid amplification reaction tube 100 at a place including the second region 112.

Here, the arrangement of the fitting section 11, the first heating section 12, and the second heating section 13 is the first arrangement. As shown in FIG. 4A, in the first arrangement, the first region 111 of the nucleic acid amplification reaction tube 100 is located in a lowermost portion of the flow channel 110 with respect to the gravitational direction. In the first arrangement, the first region 111 is located in a lowermost portion of the flow channel 110 with respect to the gravitational direction, and therefore, the reaction mixture 140 having a specific gravity larger than that of the oil 130 is located in the first region 111. In this embodiment, after the nucleic acid amplification reaction tube 100 is fitted in the fitting section 11, the fitting section 11 is covered with the lid 50, and then the elevating-type PCR apparatus 1 is activated.

Subsequently, the nucleic acid amplification reaction tube 100 is heated by the first heating section 12 and the second heating section 13. The first heating section 12 and the second heating section 13 heat different regions of the nucleic acid amplification reaction tube 100 to different temperatures. That is, the first heating section 12 heats the first region 111 to the first temperature, and the second heating section 13 heats the second region 112 to the second temperature. Accordingly, a temperature gradient in which the temperature gradually changes between the first temperature and the second temperature is formed between the first region 111 and the second region 112 of the flow channel 110. Here, a temperature gradient in which the temperature decreases from the first region 111 to the second region 112 is formed. The thermal cycling process of this embodiment is shuttle PCR, and therefore, the first temperature is set to a temperature suitable for the denaturation of a double-stranded DNA, and the second temperature is set to a temperature suitable for the annealing and elongation.

Since the arrangement of the fitting section 11, the first heating section 12, and the second heating section 13 is the first arrangement, when the nucleic acid amplification reaction tube 100 is heated, the reaction mixture 140 is heated to the first temperature. When the first period has elapsed, the main body 10 is driven by the driving mechanism 20, and the arrangement of the fitting section 11, the first heating section 12, and the second heating section 13 is switched over from the first arrangement to the second arrangement. The second arrangement is an arrangement in which the second region 112 is located in a lowermost portion of the flow channel 110 with respect to the gravitational direction. In other words, the second region 112 is a region located in a lowermost portion of the flow channel 110 with respect to the gravitational direction when the fitting section 11, the first heating section 12, and the second heating section 13 are in a predetermined arrangement different from the first arrangement. In the thermal cycler 1 of this embodiment, by the control of the control section, the driving mechanism 20 rotatively drives the main body 10. When the flanges 16 are rotatively driven by the motor by using the drive shaft as the axis of rotation, the fitting section 11, the first heating section 12, and the second heating section 13 which are fixed to the flanges 16 are rotated. Since the drive shaft is a shaft extending in the direction perpendicular to the longitudinal direction of the fitting section 11, when the drive shaft is rotated by the activation of the motor, the fitting section 11, the first heating section 12, and the second heating section 13 are rotated. In the example shown in FIGS. 4A and 4B, the main body 10 is rotated at 180°. By doing this, the arrangement of the fitting section 11, the first heating section 12, and the second heating section 13 is switched over from the first arrangement to the second arrangement.

Here, the positional relationship between the first region 111 and the second region 112 with respect to the gravitational direction is opposite from that of the first arrangement, and therefore, the reaction mixture 140 moves from the first region 111 to the second region 112 by the gravitational force. When the operation of the driving mechanism 20 is stopped after the arrangement of the fitting section 11, the first heating section 12, and the second heating section 13 has reached the second arrangement, the fitting section 11, the first heating section 12, and the second heating section 13 are held in the second arrangement. When the second period has elapsed in the second arrangement, the main body 10 is rotated again. A nucleic acid amplification reaction is performed by rotating the main body 10 while switching over between the first arrangement and the second arrangement in this manner until the number of thermal cycles has reached a predetermined number of cycles.

### Examples

Hereinafter, the invention will be described in more detail by showing Examples, however, the invention is not limited thereto.

### (1) Effect of Surfactant

In this example, with reference to the concentration of a surfactant contained in a commercially available enzyme (DNA polymerase), surfactant solutions having various concentrations were prepared, and the adhesion of each of the solutions to a tube was observed.

First, a polypropylene tube for an elevating-type PCR apparatus was filled with an oil, and then, 1.6 µL of each of the surfactant solutions was added thereto to form a liquid droplet of the surfactant solution. Then, the tube was laid on its side and left to stand for 5 minutes in a state where the liquid droplet was not in contact with the bottom. Thereafter, the tube was made to stand upright, and thereby the surfactant solution was made to move to the bottom of the tube. One minute after the surfactant solution reached the bottom, the tube was turned upside down, and the state of adhesion of the surfactant solution to the bottom of the tube was evaluated. A case where the surfactant solution immediately fell down was evaluated as "good" , and a case where even the slightest sign of adhesion of the surfactant solution to the bottom of the tube was observed, ranging from a case where the surfactant solution fell down when the tube was shaken to a case where the surfactant solution did not fall down even if the tube was shaken, was evaluated as "bad". As the oil, KF-96L-2cs which is a dimethyl silicone oil with high purity was used.

As a result, when the concentration of the surfactant was a given value or higher, the surfactant solution adhered to the bottom of the tube. In Table 1, with respect to the tube used, the highest concentration at which the surfactant solution did not adhere to the bottom of the tube is shown. That is, when the concentration of the surfactant was equal to or lower than the concentration shown in Table 1, the reaction mixture did not adhere to the inner wall, however, when the concentration of the surfactant was higher than the concentration shown in Table 1, the adhesion of the reaction mixture to the inner wall was observed.

**Table 1**

| | |
|---|---|
| NP-40 | 0.002 |
| Triton X-100 | 0.01 |
| Tween 20 | 0.1 |

| | |
|---|---|
| Unit: % (v/v) | |

When a surfactant is contained in the reaction mixture at a given concentration or higher in this manner, a phenomenon in which the reaction mixture is adhered to the inner wall of the tube and does not fall down even if the tube is turned upside down occurs.

### (2) Effect of Additive

In this Example, each of the additives shown in Table 2 was added at 5% (v/v) to KF-96L-2cs, which is a dimethyl silicone oil with high purity, followed by mixing, and the resulting mixture was used as the oil. Further, the reaction mixture was prepared as follows.

| | |
|---|---|
| Takara Ex Taq HS (x50) | 0.25 |
| 10×Ex Taq Buffer | 5.0 |
| dNTP Mixture (2.5 mM each) | 4.0 |
| InfA forward primer (20 µM) | 2.0 |
| InfA reverse primer (20 µM) | 2.0 |
| InfA probe (10 µM) | 1.0 |
| plasmid | 5.0 |
| DW | up to 50.0 (unit: µL) |

The commercially available Takara Ex Taq HS contains 0.5% NP-40 and 0.5% Tween 20, and therefore, the thus prepared reaction mixture contained 0.02% NP-40 and 0.02% Tween 20.

By using these oils and the reaction mixture, the state of adhesion of the reaction mixture to the tube was observed. The state of adhesion of the reaction mixture to the tube was evaluated in the same manner as in the above (1).

**Table 2**

| Additive | Type of additive | Evaluation |
|---|---|---|
| X-22-160AS | carbinol-modified silicone oil | good |
| X-22-3701E | carboxyl-modified silicone oil | good |
| KF-857 | amino-modified silicone oil | good |
| KF-859 | amino-modified silicone oil | good |
| KF-862 | amino-modified silicone oil | good |
| KF-867 | amino-modified silicone oil | good |
| KF-6017 | polyether-modified silicone oil | good |
| KF-8005 | amino-modified silicone oil | good |
| SR1000 | silanol-modified silicone resin | good |
| SS4230 | silanol-modified silicone resin | good |
| SS4267 | silanol-modified silicone resin | good |
| TSF4703 | amino-modified silicone oil | good |
| TSF4708 | amino-modified silicone oil | good |
| YR3370 | silanol-modified silicone resin | good |
| XF42-C5196 | amino-modified silicone oil | good |
| XF42-C5197 | amino-modified silicone oil | good |
| XS66-C1191 | fluoro-modified silicone resin | good |
| non | - | bad |

In this manner, by adding any of the additives shown in Table 2 at about 5% (v/v), the adhesion of the reaction mixture to the tube can be prevented.

## Claims

1. A biochip comprising:
a vessel;
a liquid which is contained in the vessel; and
an additive which is a carbinol-modified silicone resin, a carboxyl-modified silicone resin, an amino-modified silicone resin, a polyether-modified silicone resin, a silanol-modified silicone resin, or a fluoro-modified silicone resin,
wherein a liquid droplet of a reaction mixture which (i) has a different specific gravity from that of the liquid contained in the vessel; (ii) is immiscible with the liquid contained in the vessel; and (iii) contains a surfactant, can move in the longitudinal direction of the vessel when placed in the vessel.

2. The biochip according to claim 1, wherein the liquid is a mineral oil or a silicone oil.

3. The biochip according to claim 1 or claim 2, wherein the additive is any of X-22-160AS, X-22-3701E, KF-857, KF-859, KF-862, KF-867, KF-6017, KF-8005 (Shin-Etsu Silicone Co., Ltd.), SR1000, SS4230, SS4267, YR3370, XS66-C1191, TSF4703, TSF4708, XF42-C5196, and XF42-C5197 (Momentive Performance Materials, Inc.).

4. The biochip according to any preceding claim, wherein the concentration of the additive is 1% (v/v) or more and 50% (v/v) or less based on the total volume of the liquid and the additive.

5. The biochip according to any preceding claim, wherein the vessel is made of polypropylene.

6. The biochip according to any preceding claim, wherein the biochip comprises the liquid droplet of the reaction mixture.

7. The biochip according to claim 6, wherein the reaction mixture contains a reagent for a nucleic acid amplification reaction.

8. The biochip according to claim 6 or claim 7, wherein the surfactant in the liquid droplet is NP-40, Triton X-100, or Tween 20.

9. A nucleic acid amplification apparatus, comprising the biochip according to any preceding claim, which is fitted therein.
